(19) Europäisches Patentamt
European Patent Office
Office européen des brevets

(11) Numéro de publication: **0 000 300**
**B1**

(12) **FASCICULE DE BREVET EUROPEEN**

(45) Date de publication du fascicule du brevet: **06.01.82**

(21) Numéro de dépôt: **78400016.8**

(22) Date de dépôt: **12.06.78**

(51) Int. Cl.³: **C 07 J 1/00**, C 07 J 17/00, C 07 J 51/00, C 07 B 23/00 //A61K49/00, G01N33/58

(54) **Estra-1,3,5(10),6-tétraènes substitués, procédé de préparation et application dans la synthèse de stéroides marqués au tritium.**

(30) Priorité: **27.06.77 FR 7719613**

(43) Date de publication de la demande:
**10.01.79 Bulletin 79/1**

(45) Mention de la délivrance du brevet:
**06.01.82 Bulletin 82/1**

(84) Etats contractants désignés:
**BE CH DE GB NL SE**

(56) Documents cités:
**GB - A - 1 151 404**
**US - A - 3 020 294**

**CHEMICAL ABSTRACTS, vol. 82 (1975) 43644y & YAKUGAKU ZASSHI, 1974 94(11) 1484—8**

(73) Titulaire: **ROUSSEL-UCLAF**
**102, route de Noisy Boîte postale no.9**
**F-93230 Romainville (FR)**

(72) Inventeur: **Jouquey, Alain**
**137, rue des Pyrénées**
**F-75020-Paris (FR)**
Inventeur: **Raynaud, Jean-Pierre**
**4 villa Jocelyn**
**F-75116-Paris (FR)**
Inventeur: **Salmon, Jean**
**8, rue du Général de Castelnau**
**F-75015-Paris (FR)**

(74) Mandataire: **Markovic, Borivoj**
**102, route de Noisy Boîte Postale no 9**
**F-93230-Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 000 300 B1

Estra-1,3,5(10),6-tétraènes substitués, procédé de préparation et application dans la synthèse de stéroides marqués au tritium

La présente invention concerne de nouveaux stéroïdes de la série des estratétraènes substitués, leur procédé de préparation et leur application à la synthèse de dérivés de la série des estradiols marqués au tritium.

L'état de la technique révèle les documents suivants:

— la publication de Chemical Abstracts, Vol 82 (1975), 43644Y et YAKUGAKU ZASSHI, 1974, 94 (11) 1484—8, a relative à la préparation du 2-hydroxy estra-1, 3, 5 (10)-trien 17$\beta$-ol [6,7—$^3$H] au moyen de tritium gazeux à partir du dérivé 2—OR estra-1, 3, 5 (10), 6-tetraen 17$\beta$—$OR_1$ correspondant;

— le brevet britannique 1 151 404 relatif aux 11$\beta$-alcoxy 13$\beta$-alkyl gona-1, 3, 5 (10) triènes et leur préparation lors de laquelle le produit$\Delta^{4,9}$ correspondant est aromatisé par l'isomérisation catalytique;

— le brevet US 3 020 294 relatif à un dibromo$\Delta^4$-3-ceto stéroïde, susceptible d'être de hidrohalogène pour former le $\Delta^{1,4,6}$-3-céto stéroïde correspondant, dont on aromatise ensuite le noyau A par une hydrogénation catalytique.

L'invention a pour objet les stéroïdes de formule générale I

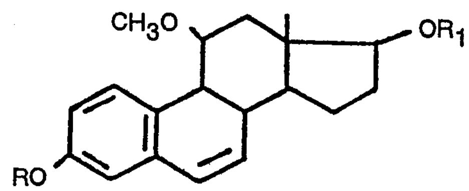

I

dans laquelle R représente de l'hydrogène ou R', R' étant un radical acyle ayant de 1 à 5 atomes de carbone, le radical benzoyle substitué ou non, ou un radical tétrahydropyranyle, trityle ou triméthylsilyle, et $R_1$ représente un radical acyle ayant de 1 à 5 atomes de carbone, le radical benzoyle substitué ou non ou un radical tétrahydropyranyle, trityle ou triméthylsilyle.

Lorsque R et/ou $R_1$ représente un radical acyle ayant de 1 à 5 atomes de carbone, il peut être par exemple le reste d'acides formique, acétique, propionique, butyrique ou valérique.

Lorsque R et/ou $R_1$ représente un radical benzoyle substitué, il peut être substitué par exemple par un halogène tel que le chlore ou le brome, par un radical alcoyle ayant de 1 à 5 atomes de carbone, par le radical trifluorométhoxy, ou par un radical alcoyloxy ayant de 1 à 5 atomes de carbone.

Parmi les produits de formule I, on citera notamment:

—l'acétate 17$\beta$ de méthoxy-11$\beta$ estra-1, 3, 5 (10), 6-tetraen diol-3, 17$\beta$

—le diacétate de méthoxy-11$\beta$ estra-1, 3, 5 (10), 6-tetraen diol-3, 17$\beta$.

Les produits de formule générale I sont utiles en tant que produits de départ pour la synthèse de dérivés stéroïdes substitués marqués au tritium.

L'invention a également pour objet un procédé de préparation des produits répondant à la formule générale I ci-dessus.

Ce procédé est caractérisé en ce que l'on:

—bloque la fonction hydroxyle en position 17$\beta$ de la 11$\beta$-méthoxy nor-testostérone au moyen d'un agent d'estérification ou d'éthérification.

—soumet la $OR_1$—17$\beta$ méthoxy-11$\beta$ nor-testostérone obtenue, $R_1$ ayant ici et dans ce qui suit les significations précitées, à l'action d'un agent d'halogénation.

—fait agir sur le $OR_1$—17$\beta$ dihalogéno-2,6 méthoxy-11$\beta$ estren-4 one-3 résultant un agent de déshalohydratation pour obtenir le $OR_1$—17$\beta$ méthoxy-11$\beta$ estra-1,3,5(10),6-tetraen ol-3 cherché, dont on bloque, le cas échéant, la fonction hydroxyle en 3 au moyen d'un agent d'estérification ou d'éthérification pour obtenir le OR'—3, $OR_1$—17$\beta$ méthoxy-11$\beta$ estra-1,3,5(10),6-tetraene, R' ayant les significations précitées.

Dans les conditions préférentielles de mise en oeuvre, le procédé de l'invention est caractérisé en ce que:

—l'agent d'estérification au moyen duquel on bloque la fonction hydroxyle en position 17 ou en position 3 est un acide ou un dérivé fonctionnel d'acide; ce dérivé fonctionnel peut être l'anhydride acétique ou le chlorure de benzoyle;

—l'agent d'étherification au moyens duquel on bloque la fonction hydroxyle en position 17 ou en position 3 est le dihydropyran, le chlorure de trityle ou le chlorure de triméthylsilyle;

—l'agent d'halogénation à l'action duquel on soumet la $OR_1$—17$\beta$ méthoxy-11$\beta$ nor-testérone est le brome dans l'acide acétique;

—l'agent de déshalohydratation que l'on fait agir sur le $OR_1$—17$\beta$ dihalogéno-2,6 méthoxy-11$\beta$ estren-4-one-3 est le chlorure de lithium dans le diméthyl formamide et on opère sous atmosphère inerte.

Au cours de l'exécution de ce procédé, les produits industriels nouveaux suivants ont été préparés:

—l'acétate de méthoxy-11$\beta$ nor-testostérone

—l'acétoxy-17β dibromo-2,6 méthoxy-11β estren-4 one-3.

Comme déjà indiqué les produits de formule générale I sont les produits de départ pour la synthèse de dérivés stéroïdes marqués au tritium. Leur application en tant que tels est également un objet de la présente invention et est caractérisée en ce que l'on:

—réduit un produit de formule générale I, dans laquelle R et $R_1$ ont les significations précitées, par action d'hydrogène tritié en présence d'un catalyseur,

—soumet le /6, 7 $^3H$/ OR—3 $OR_1$—17β méthoxy-11β estra-1,3,5(10)-triene à l'action d'une base forte, ou d'un acide faible en milieu aqueux, ou d'un hydracide, pour obtenir le /6,7—$^3H$/ me"thoxy-11β estra-1,3,5(10)-trien diol-3,17β que l'on oxyde, selon la réaction d'Openauer, en /6,7—$^3H$/ hydroxy-3 méthoxy-11β estra-1,3,5(10)-trien one-17, au moyen de cyclohéxanone en présence d'isopropylate d'aluminium;

—soumet ce dernier à l'action d'un agent d'éthynylation, en présence de ter-amylate de sodium dans le toluène et obtient le /6,7—$^3H$/ méthoxy-11β éthynyl-17α estradiol.

Dans les conditions préférentielles de mise en oeuvre, cette application est caractérisée en ce que:

—le catalyseur est l'hydroxyde de palladium,

—la base forte est la soude et on opère dans le méthanol,

—l'acide aqueux faible est l'acide acétique,

—l'hydracide est l'acide chlorhydrique,

—et l'agent d'éthynylation est l'acétylène.

Au cours de l'exécution de ce procédé d'application les produits industriels nouveaux suivants ont été préparés:

—le /6,7—$^3H$/ diacétate de méthoxy-11β estra-1,3,5(10)-trien diol-3,17β

—le /6,7—$^3H$/ méthoxy-11β estra-1,3,5(10)-trien diol-3,17β

—le /6,7—$^3H$/ hydroxy-3 méthoxy 11β estra 1,3,5(10)-trien one-17.

Parmi ces produits, le /6,7—$^3H$/ méthoxy-11β estra-1,3,5(10)-trien diol-3,17β et le /6,7—$^3H$/ hydroxy-3 méthoxy-11β estra-1,3,5(10)-trien one-17, présentent un intérêt particulier.

Ces produits conduisent notamment au /6,7—$^3H$/ méthoxy-11β éthynyl-17α estradiol qui présente un activité spécifique de l'ordre de 52 Ci/mM.

Ce dernier produit permet l'étude et le dosage du récepteur spécifique d'estrogène présent dans les cellules des tissus ou organes cibles de l'action des estrogènes, comme l'utérus, le vagin, l'hypophyse, l'hypotalamus et les tumeurs, comme par exemple du sein, de la prostate ou de l'adénome, aussi bien chez l'animal que chez l'homme. En effet ce produit ne se fixe pas avec les protéines plasmatiques liant les hormones telles que la testostérone et l'estradiol, chez la femme c'est un marqueur de choix du récepteur tissulaire des estrogènes avec lequel il forme un complexe de forte affinité et de grande stabilité, d'autant qu'il n'a aucune interaction avec les récepteurs tissulaires des autres classes d'hormones stéroidiennes (glucocorticoïdes, minéralocorticoïdes, androgènes ou progestogènes).

Enfin, il permet le dosage du méthoxy-11β éthynyl-17α estradiol dans le plasma ou d'autres liquides biologiques par radioimmuno essai.

L'utilisation du /6,7—$^3H$/ méthoxy-11β éthynyl-17α estradiol a été décrite par J. P. RAYNAUD et coll., "Progesterone receptors in normal and Neoplastic Tissues", ed. W. L. McGUIRE et Al., Raveh Press, New York, 1977, p.p. 171—191.

Le produit de départ du procédé de préparation des produits de formule générale I, le 11β-méthoxy nor-testostérone a été décrit dans le brevet français 2 115 033.

Les exemples suivants illustrent à titre non limitatif la mise en oeuvre de l'invention.

### Exemple I
### Diacétate du méthoxy-11β estra-1,3,5(10), 6-tétraène diol-3-17β

*Stade A: Méthoxy-11β-acétoxy-17β nor-testostérone*

On dissout 15 g de méthoxy-11β nor-testostérone dans 75 cm3 de pyridine et 37,5 cm3 d'anhydride acétique. On agite 13 heures à température ambiante. Puis, on ajoute 350 cm3 d'eau et de glace. Après 30 minutes d'agitation à 0°+5°C, on essore et on lave avec de l'eau glacée. On sèche sous vide à température ambiante et on obtient 16,7 g du produit désiré fondant à +140°C. Ce produit est recristallisé dans l'éther isopropylique.

| | | | |
|---|---|---|---|
| Point de fusion | : +140°C | | |
| Analyse | : $C_{21}H_{30}O_4$ | | |
| Calculé | : C% 72,80 | H% 8,73 | 0% 18,47 |
| Trouvé | : 72,9 | 8,5 | |

/α/$_D$ =+58°35 (C=1,2% dans l'éthanol)

*Stade B: Acétoxy-17β dibromo-2,6 méthoxy-11β estrène-4 one-3*

On dissout 11,7 g du produit obtenu au stade A dans 117 cm3 d'éther anhydre. En maintenant la température à 20°C on ajoute, en 30 minutes, 10,78 g de brome en solution dans 58,5 cm3 d'acide acétique. On agite encore 15 minutes puis on chasse l'éther sous vide à 30°C. L'extrait sec est repris par 250 ml d'eau. On extrait la phase aqueuse par du chlorure de méthylène, lave la phase organique avec une solution saturée de bicarbonate et ensuite par de l'eau, la sèche sur sulfate de sodium anhydre. Cette solution d'acétoxy-17β dibromo-2,6 méthoxy-11β estrène-4 one-3 dans le chlorure de méthylène est utilisée telle quelle pour le stade suivant de la synthèse.

*Stade C: Acétate-17β méthoxy-11β estra-1,3,5(10), 6 tetraène diol-3, 17β*

On mélange 10,53 g de chlorure de lithium et 117 cm3 de diméthyl formamide. On chauffe sous agitation et sous azote jusqu'à 110—115°C puis on ajoute la solution chlorométhylénique obtenue au stade précédent. On chasse le chlorure de méthylène par distillation puis on laisse 2 heures à 110—115°C sous agitation et sous azote. On refroidit à +20°C et on verse sour 500 cm3 d'eau et de glace. La gomme obtenue est extraite par du chlorure de méthylène. On lave la phase organique avec une solution saturée de bicarbonate de sodium puis à l'eau. On sèche sur sulfate de sodium. Après évaporation à sec sous vide à +30°C, on obtient un résidu que l'on purifie par chromatographie sur silice, (éluant benzène 9 acétate d'éthyle 1). Le produit obtenu est lavé au benzène et séché.

On obtient 4,267 g de produit cherché.

F. =+189°C.

Les eaux mères reprises par du benzène donnent un 2ème jet de 860 mg de produit fondant à +189°C.

*Stade D; Diacétate du méthoxy-11β estra-1,3,5(10), 6 tétraène diol-3, 17β*

On dissout 4,1 g de produit obtenu au stade précédent dans 20 cm3 de pyridine et 10 cm3 d'anhydride acétique. On agite à 20°C pendant 16 heures puis on ajoute 100 cm3 d'eau et de glace. La gomme formée est extraite au chlorure de méthylène. On lave la phase organique à l'acide chlorhydrique N puis par une solution saturée de bicarbonate de sodium et enfin par de l'eau. On sèche sur sulfate de sodium anhydre puis on évapore à sec sous vide. On obtient 4,49 g d'une huile incolore.

*Analyse*

Spectre UV

*1° dans l'éthanol*

| | | | | |
|---|---|---|---|---|
| max. | 220 nm | $E^{1\%}_{1\,cm}$ | 712 | ε=27.400 |
| infl. vers | 225 nm | | 656 | |
| max. | 263 nm | | 223 | ε=8.600 |
| infl. vers | 269—270 nm | | 145 | |
| infl. vers | 289 nm | | 43 | |
| max. | 300 nm | | 25 | ε=960 |

*2° dans la soude éthanolique N/10*

| | | | | |
|---|---|---|---|---|
| max. | 241 nm | $E^{1\%}_{1\,cm}$ | 776 | ε=29.800 |
| infl. vers | 267 nm | | 124 | |
| max. | 328 nm | | 57 | ε=2.200 |

Exemple II

/6,7—$^3$H/ méthoxy-11β éthynyl-17α estradiol

*Stade A: Diacétate du /6,7—$^3$H/ méthoxy-11β estra-1,3,5(10) triène diol 3, 17β*

On refroidit par l'azote liquide le mélange réactionnel constitué par 45,2 mg de diacétate de méthoxy-11β estra-1,3,5(10),6 tétraène diol-3, 17β, 10 mg de noir palladié et 0,6 cm3 d'acétate d'éthyle redistillé, puis introduit sous vide 3,87 cm3 de tritium, mesurés à 0°C et à pression normale d'une activité totale de 10 Ci. Puis on laisse revenir le mélange réactionnel à température ambiante, maintient sous agitation pendant 3 heures, récupère l'excès de tritium, filtre, distille sous pression réduite le solvant et, après élimination du tritium labile, obtient 46 mg de diacétate du /6,7—$^3$H/ méthoxy-11β estra-1,3,5(10) triène dio-3,17β cherché d'une activité spécifique de 54,03 Ci/mM.

*Stade B: /6,7—$^{3}H$/ méthoxy-11β estra-1,3,5(10) triène diol-3, 17β*

On agite sous courant d'azote 41 mg du produit obtenu au stade précédent, 4 cm3 de méthanol redistillé, 0,5 cn3 d'eau et 0,5 cm3 de lessive de soude 36°Bé. On porte au reflux sous agitation et sous azote pendant 2 heures. Après refroidissement à 20°C on ajoute 0,5 cm3 d'acide acétique et 1,5 cm3 d'eau. Le produit cristallise. On glace 1 heure puis on essore et lave à l'eau glacée. On sèche sous vide en présence d'anhydride phosphorique. On obtient 30,6 mg de prodiut cherché.

F =+245°C

Activité spécifique 52 Ci/mM.

*Stade C: /6,7—$^{3}H$/ hydroxy-3 méthoxy-11β estra-1,3,5(10) triène one-17*

On amène à 50°C, sous argon, 20,9 mg du produit obtenu au stade precédent avec 5 cm3 de toluène et 2 cm3 de cyclohexanone. On distille sous passage d'argon 1 cm3 de toluène. Puis on introduit, en 30 minutes au total, 4 fois 1 cm3 d'une solution d'isopropylate d'aluminium dans 50 cm3 de toluène, en distillant au fur et à mesure, de façon à maintenir le volume constant. On laisse encore 15 minutes au reflux sous argon. Ensuite on introduit, en distillant au fur et à mesure, une solution de 1 g de tartrate de sodium et de potassium dans 10 cm3 d'eau. On entraîne les solvants à la vapeur d'eau. On refroidit dans un bain de glace. Le produit cristallise. On agite 30 minutes à 0° avant d'essorer. On lave à l'eau, on sèche sous vide en présence d'anhydride phosphorique à température ambiante. On obtient 18,8 mg de produit brut que l'on utilise tel quel pour le stade suivant de la synthèse.

Stade D: */6,7—$^{3}H$/ méthoxy-11β éthynyl-17α estradiol*

Dans 10 cm3 d'une solution de téramylate de sodium à 2,8 g de Na pour 100 cm3 on fait barboter de l'acétylène pendant 2 heures. Puis on ajoute dans le produit obtenu au stade précédent dissous dans 2,5 cm3 de tétrahydrofurane. On maintient le barbotage d'acétylène pendant 5 heures sous agitation et à température ambiante. On remplace le barbotage d'acétylène par de l'argon et on acidifie immédiatement, en refroidissant dans un bain de glace, par 2 cm3 d'acide acétique à 50% d'eau. On extrait à l'éther et on lave à l'eau jusqu'à neutralité. On sèche sur sulfate de sodium. Cette solution est concentrée sous vide. On obtient 25 mg de produit que l'on dissout dans 1 cm3 de tétrahydrofurane pur, pour faire une chromatographie sur plaque, avec, comme solvant de migration, le système benzène 7 acétate d'éthyle 3. Le produit est isolé par élution avec un mélange chlorure de méthylène 1 acétone 1. On concentre sous vide et on obtient 11,1 mg de produit solvaté à 26%, soit 8,17 mg en produit non solvaté.

Activité spécifique: 52 Ci/mM.

**Revendications**

1. Nouveaux stéroïdes de formule générale I:

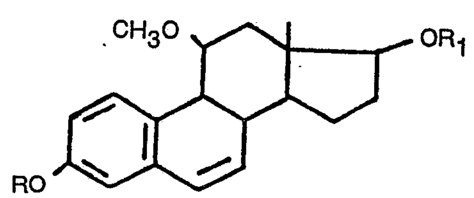

I

dans laquelle R représente de l'hydrogène ou R', R' étant un radical acyle ayant de 1 à 5 atomes de carbone, le radical benzoyle substitué ou non, ou un radical tétrahydropyranyle, trityle ou triméthylsilyle, et $R_1$ représente un radical acyle ayant de 1 à 5 atomes de carbone, le radical benzoyle substitué ou non, ou un radical tétrahydropyranyle, trityle ou triméthylsilyle.

2. L'acétate-17β de méthoxy-11β estra-1,3,5(10), 6 tétraen diol-3,17β.

3. Le diacétate de méthoxy-11β estra-1,3,5(10), 6 tétraen diol-3,17β.

4. Procédé de préparation des produits tels que définis aux revendications 1, 2 ou 3, caractérisé en ce que l'on:

—bloque la fonction hydroxyle en position 17β de la 11β-méthoxy nor-testostérone au moyen d'un agent d'estérification ou d'éthérification,

—soumet la $OR_1$—17β méthoxy-11β nor-testostérone obtenue, $R_1$ ayant ici et dans ce qui suit les significations précitées, à l'action d'un agent d'halogénation.

—fait agir sur le $OR_1$—17β dihalogéno-2,6 méthoxy-11β estren-4-one-3 résultant un agent de deshalohydratation pour obtenir le $OR_1$—17β méthoxy-11β estra-1,3,5(10),6-tétraen ol-3 cherché, dont on bloque, le cas échéant, la fonction hydroxyle en 3 au moyen d'un agent d'estérification ou d'éthérification pour obtenir le OR'—3, $OR_1$—17β méthoxy-11β estra, 1,3,5(10),6-tétraene, R' ayant les significations précitées.

5. Application des produits tels que définis aux revendications 1, 2 et 3 à la synthèse de composés stéroïdes marqués au tritium, caractérisée en ce que l'on:

—réduit un produit de formule générale I, dans laquelle R et $R_1$ ont les significations précitées, par action d'hydrogène tritié en présence d'un catalyseur,
—soumet le /6,7—$^3H$/ OR—3 $OR_1$—17β méthoxy-11β estra-1,3,5(10)-triene résultant à l'action d'une base forte, ou d'un acide faible en milieu aqueux, ou d'un hydracide, pour obtenir le /6,7—$^3H$/ méthoxy-11β estra-1,3,5(10)-trien diol-3, 17β, que l'on oxyde en /6,7—$^3H$/hydroxy-3 méthoxy-11β estra-1,3,5(10)-trien one-17 au moyen de cyclohexanone en présence d'isopropylate d'aluminium,
—soumet ce dernier à l'action d'un agent d'éthynylation, en présence de ter-amylate de sodium dans le toluène et obtient le /6,7—$^3H$/ méthoxy-11β éthynyl-17α estradiol.

6. Application selon la revendication 5, caractérisée en ce que:
—le catalyseur est l'hydroxyde de palladium,
—la base forte est la soude et l'on opère dans le méthanol,
—l'acide aqueux faible est l'acide acétique,
—l'hydracide est l'acide chlorhydrique
—et l'agent d'éthynylation est l'acétylène.

**Patentansprüche**

1. Neue Steroide der allgemeinen Formel I

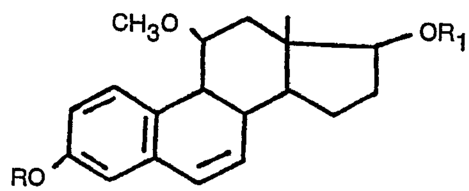

I

worin R ein Wasserstoffatom oder R' darstellt, wobei R' ein Acylrest mit 1 bis 5 Kohlenstoffatomen, ein substituierter oder unsubstituierter Benzoylrest oder ein Tetrahydropyranylrest, ein Tritylrest oder ein Trimethylsilylrest ist und $R_1$ einen Acrylrest mit 1 bis 5 Kohlenstoffatomen, einen substituierten oder unsubstituierten Benzoylrest oder einen Tetrahydropyranylrest, einen Tritylrest oder Trimethylsilylrest bedeutet.

2. Das 17β-Acetat von 11β-Methoxy-östra-1,3,5(10),6-tetraen-3,17β-diol.

3. Das Diacetat von 11β-Methoxy-östra-1,3,5(10),6-tetraen-3,17β-diol.

4. Verfahren zur Herstellung von Produkten gemäß Anspruch 1, 2 oder 3, dadurch gekennzeichnet, daß man die Hydroxylfunktion in 17β-Stellung des 11β-Methoxy-nor-testosterons mit Hilfe eines Veresterungs- oder Verätherungsmittels blockiert, das erhaltene 17β-$OR_1$-11β-Nor-testosteron, worin $R_1$ hier und im folgenden die vorstehend angegebenen Bedeutungen besitzt, der Einwirkung eines Halogenierungsmittels unterzieht, auf das erhaltene 17β-$OR_1$-2,6-Dihalogeno-11β-methoxy-östr-4-en-3-on ein Halogenwasserstoffabspaltungsmittel einwirken läßt, um das gewünschte 17β—$OR_1$-11β-Methoxy-östra-1,3,5(10), 6-tetraen-3-ol zu erhalten, an dem man gegebenenfalls die Hydroxylfunktion in 3-Stellung mit Hilfe eines Versterungs- oder Verätherungsmittels blockiert, um das 3—OR'-17β-$OR_1$-11β-Methoxy-östra-1,3,5(10),6-tetraen zu erhalten, worin R' die vorstehend angegebenen Bedeutungen besitzt.

5. Verwendung der Produkte gemäß den Ansprüchen 1, 2 und 3 für die Synthese von tritiummarkierten Steroidverbindungen, dadurch gekennzeichnet, daß man ein Produkt der allgemeinen Formel I, worin R und $R_1$ die vorstehend angegebenen Bedeutungen besitzen, durch Einwirkung von tritiertem Wasserstoff in Gegenwart eines Katalysators reduziert, das erhaltene [6,7—$^3H$]-3OR-17β-$OR_1$-11β-Methoxy-östra-1,3,5(10)-trien der Einwirkung einer starken Base oder einer schwachen Säure in wäßrigem Milieu oder einer Wasserstoffsäure unterzieht, um das [6,7—$^3H$]-11β-Methoxy-östra-1,3,5(10)-trien-3,17β-diol zu erhalten, das man zum [6,7—$^3H$]-3-Hydroxy-11β-methoxy-östra-1,3,5(10)-trien-17-on mit Hilfe von Cyclohexanon in Gegenwart von Aluminiumisopropylat oxidiert, dieses letztere der Einwirkung eines Äthinylierungsmittels in Gegenwart von Natrium-tert-amylat in Toluol unterzieht und das [6,7—$^3H$]-11β-Methoxy-17α-äthinyl-östradiol erhält.

6. Verwendung gemäß Anspruch 5, dadurch gekennzeichnet, daß
der Katalysator Palladiumhydroxyd ist,
die starke Base Natronlauge ist, und man in Methanol arbeitet,
die schwache wäßrige Säure Essigsäure ist,
die Wasserstoffsäure Chlorwasserstoffsäure ist, und das Äthinylierungsmittel Acetylen ist.

**Claims**

1. New steroids of general formula I:

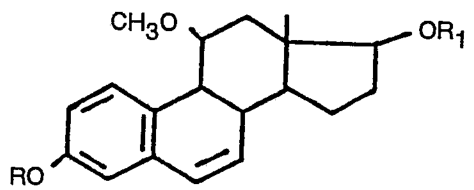

I

in which R represents hydrogen or R', R' being an acyl radical having from 1 to 5 carbon atoms, the substituted or unsubstituted benzoyl radical or a tetrahydropyranyl, trityl or trimethylsilyl radical and $R_1$ represents an acyl radical having from 1 to 5 carbon atoms, the substituted or unsubstituted benzoyl radical or a tetrahydropyranyl, trityl or trimethylsilyl radical.

2. 11β-methoxy estra-1,3,5(10),6 tetraene 3,17β-diol acetate.

3. 11β-methoxy estra-1,3,5(10),6 tetraene 3,17β-diol diacetate.

4. Process for preparing the products as defined in Claims 1, 2 or 3, characterised in that:
—the hydroxyl function at position 17β of the 11β-methoxy nortestosterone is blocked by means of an esterification or etherification agent,
—the 17β—$OR_1$ 11β-methoxy nor-testosterone obtained, $R_1$ having here and in the following the aforementioned meanings, is subject to the action of a halogenation agent and
—a dehydrohalogenation agent is reacted with the resultant 17β—$OR_1$ 2,6-dihalogeno 11β-methoxy estr-4-en 3-one to obtain the 17β—$OR_1$ 11β-methoxy estra-1,3,5(10),6-tetraen 3-ol sought of which the hydroxyl function at 3 is blocked, if need be, by means of an esterification or etherification agent to obtain 3—OR', 17β—$OR_1$ 11β-methoxy estra-1,3,5(10),6-tetraene, R' having the aforementioned meanings.

5. Use of the products as defined in claims 1, 2 and 3 in the synthesis of steroid compounds labelled with tritium, characterised in that:
— a product of general formula I, in which R and $R_1$ have the aforementioned meanings, is reduced by the action of tritiated hydrogen in the presence of a catalyst,
— the resultant [6,7—$^3H$] 3-OR 17β—$OR_1$ 11β-methoxy estra-1,3,5(10)-triene is subjected to the action of a strong base or of a weak acid in aqueous medium or of a hydracid to obtain [6,7—$^3H$] 11β-methoxy estra-1,3,5(10)-triene 3,17β-diol which is oxidised into [6,7—$^3H$] 3-hydroxy 11β-methoxy estra-1,3,5(10)-trien 17-one by means of cyclohexanone in the presence of aluminium isopropylate
— the latter is subjected to the action of an ethynylation agent in the presence of sodium teramylate in toluene and [6,7—$^3H$] 11β-methoxy 17α-ethynyl estra diol is obtained.

6. Use according to Claim 5, characterised in that:
— the catalyst is palladium hydroxide,
— the strong base is sodium hydroxide and work is carried out in methanol,
— the weak aqueous acid is acetic acid,
— the hydracid is hydrochloric acid,
— and the ethynylation agent is acetylene.